# EUROPÄISCHE PATENTSCHRIFT

(11) **EP 3 004 200 B1**
(45) Veröffentlichungstag und Bekanntmachung des Hinweises auf die Patenterteilung: **29.03.2017**
(21) Anmeldenummer: 14724740.7
(22) Anmeldetag: 16.05.2014
(51) Int. Cl.: C08G 18/67, C08G 18/68, C08G 18/22, C09D 175/16

(54) **VERFAHREN ZUR HERSTELLUNG VON URETHAN(METH)ACRYLATEN**
METHOD FOR PRODUCING URETHANE (METH)ACRYLATES
PROCÉDÉ DE PRODUCTION D'URÉTHAN(MÉTH)ACRYLATES

(30) Priorität: 27.05.2013 EP 13169357
(43) Veröffentlichungstag der Anmeldung: 13.04.2016
(73) Patentinhaber: BASF SE, 67056 Ludwigshafen am Rhein (DE)
(72) Erfinder: SCHWALM, Reinhold, 67157 Wachenheim (DE); NEUMANN, Susanne, 67346 Speyer (DE); KIMPEL, Delphine, 67346 Speyer (DE)
(74) Vertreter: Reinhardt, Jürgen
(86) Internationale Anmeldenummer: PCT/EP2014/060059
(87) Internationale Veröffentlichungsnummer: WO 2014/191228

(56) Entgegenhaltungen:
- US-A- 4 188 472
- US-A1- 2004 157 995

## Beschreibung

Die vorliegende Erfindung beschreibt ein neues Verfahren zur Herstellung von Urethan(meth)acrylaten.

Urethanacrylate auf Basis von caprolactonmodifizierten Harzen sind z.B. aus der US 4,188,472 bekannt. In DE 2939584 (=US4188472) werden 2-Hydroxyethylacrylat mit epsilon-Caprolacton ringöffnene in Gegenwart verschiedener Katalysatoren auf Basis Titan oder Zinn oder organischer Säuren (Schwefelsäure, p-Toluolsulfonsäure) miteinander umgesetzt und das entstandene Produkt anschließend mit Diisocyanaten zum Urethan umgesetzt.

Aus US 7022778 ist es bekannt, 2-Hydroxyethylacrylat mit epsilon-Caprolacton in Gegenwart von Zink Octoat umzusetzen. Das Reaktionsprodukt wird als Reaktivverdünner in Zweikomponenten Polyurethanbeschichtungsmassen verwendet.

Nachteilig an diesen Reaktionsführungen ist es, daß die offenbarten Katalysatoren zu einer uneinheitlichen Verteilung des Anlagerungsproduktes führen.

Die eingesetzten zinnorganischen Katalysatoren führen zu einer bestimmten Molekulargewichtsverteilung bei der Umsetzung von Hydroxyalkylacrylaten mit Caprolacton. Bei dieser Reaktion bleibt einerseits nicht umgesetztes Hydroxyalkylacrylat zurück und andererseits entstehen Umsetzungsprodukte von Hydroxyalkylacrylaten mit bis zu 10 Caprolactoneinheiten. Da Produkte mit 3 und mehr Caprolactoneinheiten jedoch zum Kristallisieren neigen, sollte deren Konzentration nicht zu hoch sein. Ferner ergeben die Umsetzungsprodukte der nichtumgesetzten Hydroxyalkylacrylate mit Diisocyanaten nur wenig flexible Urethanacrylate.

Um dieser Forderung gerecht zu werden, sollte die Viskosität der ersten Stufe, d.h. der Umsetzung von Hydroxyethylacrylat mit 2 Mol Caprolacton in einem Viskositätsbereich von 60 - 90 mPas (gemessen mit Epprecht Kegel-/Plattenviskosimeter (Cone B) bei 23 °C liegen.

Aus US 2011/039971 A1 ist es bekannt, ein kommerziell verfügbares Anlagerungsprodukt von epsilon-Caprolacton an 2-Hydroxyethylacrylat mit weiteren Komponenten an trifunktionelle Polyisocyanate zum Urethan umzusetzen.

Als Katalysatoren für die Urethanbildung werden dabei in allgemeiner Form Dibutyl zinn laurat, Wismut carboxylat oder Zirkonium chelate erwähnt. Es bleibt offen, welcher Katalysator explizit in den Beispielen eingesetzt wird.

Aus US 6534128 ist es bekannt, ein kommerziell verfügbares Anlagerungsprodukt von epsilon-Caprolacton an 2-Hydroxyethylacrylat mit weiteren Komponenten an Diisocyanate zum Urethan umzusetzen.

Als Katalysatoren für die Urethanbildung werden dabei in allgemeiner Form Dibutyl zinn dilaurat, andere organische Zinnverbindungen, Organowismut und Organozirkoniumverbindungen erwähnt. In den Beispielen wird als Katalysator ausschließlich Dibutyl zinn dilaurat eingesetzt.

Nachteilig an diesen Rekationsführungen ist es, daß aus der Herstellung des Anlagerungsproduktes noch Spuren des Katalysators im kommerziell verfügbaren Produkt enthalten sind und dann für die Bildung des Urethans zusätzlich noch weiterer Katalysator zur Reaktion zugegeben wird.

Aufgabe der vorliegenden Erfindung war es, eine Reaktionsführung zur Herstellung derartiger Urethan(meth)acrylate zu entwickeln, in der das Anlagerungsprodukt aus Lacton und Hydroxyalkyl(meth)acrylat in einheitlicherer Form als im Stand der Technik erhalten wird, um eine erhöhte Flexibilität z.B. für Anwendungen auf flexiblen Substraten zu erhalten. Dabei soll ein Katalysator eingesetzt werden, der auch im Folgeschritt für die Herstellung des Urethans eingesetzt werden kann.

Die Aufgabe wurde gelöst durch ein Verfahren zur Herstellung von Urethan(meth)acrylaten der Formel 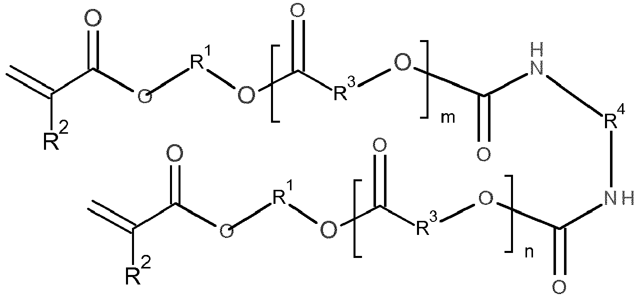 in dem man in einem ersten Schritt ein Hydroxyalkyl(meth)acrylat (A) der Formel 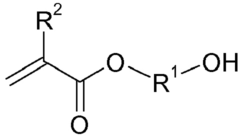 mit einem Lacton (B) der Formel 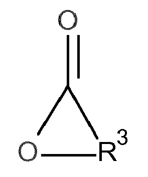 in Gegenwart mindestens einer Zink- und/oder Wismutverbindung (C) miteinander umsetzt,

und in einem weiteren Schritt das so erhaltene zink- bzw. bismuthaltige Produkt aus dem ersten Schritt mit mindestens einem cycloaliphatischen oder unsymmetrischen aliphatischen Diisocyanat (D) umsetzt.

In der obigen Formel bedeuten
- R¹: einen zweiwertigen, 2 bis 12 Kohlenstoffatome aufweisenden Alkylenrest, der gegebenenfalls mit C₁- bis C₄-Alkylgruppen substituiert und/oder durch ein oder mehrere Sauerstoffatome unterbrochen sein kann, bevorzugt 2 bis 10 Kohlenstoffatome aufweisend, besonders bevorzugt 2 bis 8 und ganz besonders bevorzugt 3 bis 6 Kohlenstoffatome aufweisend,
- R²: jeweils unabhängig voneinander Methyl oder Wasserstoff, bevorzugt Wasserstoff,
- R³: einen zweiwertigen, 1 bis 12 Kohlenstoffatome aufweisenden Alkylenrest, der gegebenenfalls mit C₁- bis C₄-Alkylgruppen und/oder durch ein oder mehrere Sauerstoffatome unterbrochen substituiert sein kann, bevorzugt 2 bis 10, besonders bevorzugt 3 bis 8 und ganz besonders bevorzugt 3 bis 4 Kohlenstoffatome aufweisend, und
- n: und m unabhängig voneinander positive Zahlen von 1 bis 5, bevorzugt 2 bis 5, besonders bevorzugt 2 bis 4, ganz besonders bevorzugt 2 bis 3 und insbesondere 2 bis 2,5.
- R⁴: bedeutet dabei einen zweiwertigen organischen Rest dar, der durch Abstraktion beider Isocyanatgruppen aus einem cycloaliphatischen oder unsymmetrischen aliphatischen Diisocyanat entsteht. Beispiele für derartige cycloaliphatische oder unsymmetrische aliphatische Diisocyanat sind unten angegeben.

Die Werte für n und m können im statistischen Mittel auch ungeradzahlige Werte annehmen, sind dann aber natürlich bezogen auf jedes einzelne Molekül der obigen Formel geradzahlig.

C₁-C₄-Alkyl bdeutet im Rahmen dieser Schrift Methyl, Ethyl, n-Propyl, *iso*-Propyl, n-Butyl, *iso-*Butyl, *sek-*Butyl oder *tert*-Butyl, bevorzugt Methyl, Ethyl und n-Butyl und besonders bevorzugt Methyl.

Beispiele für den Rest R¹ sind 1,2-Ethylen, 1,2- oder 1,3-Propylen, 1,2-, 1,3- oder 1,4-Butylen, 1,1-Dimethyl-1,2-ethylen, 1,2-Dimethyl-1,2-ethylen, 1,5-Pentylen, 1,6-Hexylen, 1,8-Octylen, 1,10-Decylen oder 1,12-Dodecylen. Bevorzugt sind 1,2-Ethylen, 1,2- oder 1,3-Propylen, 1,4-Butylen und 1,6-Hexylen, besonders bevorzugt sind 1,2-Ethylen, 1,2-Propylen und 1,4-Butylen, ganz besonders bevorzugt ist 1,2-Ethylen.

Beispiele für den Rest R³ sind Methylen, 1,2-Ethylen, 1,2-Propylen, 1,3-Propylen, 1,2-Butylen, 1,3-Butylen, 1,4-Butylen, 1,5-Pentylen, 1,5-Hexylen, 1,6-Hexylen, 1,8-Octylen, 1,10-Decylen, 1,12-Dodecylen, 2-Oxa-1,4-butylen, 3-Oxa-1,5-pentylen oder 3-Oxa-1,5-hexylen, bevorzugt sind 1,3-Propylen, 1,4-Butylen, 1,5-Pentylen, 1,5-Hexylen und 1,12-Dodecylen, besonders bevorzugt ist 1,5-Pentylen.

Bevorzugte Urethan(meth)acrylate der vorliegenden Erfindung sind dabei solche der Formel 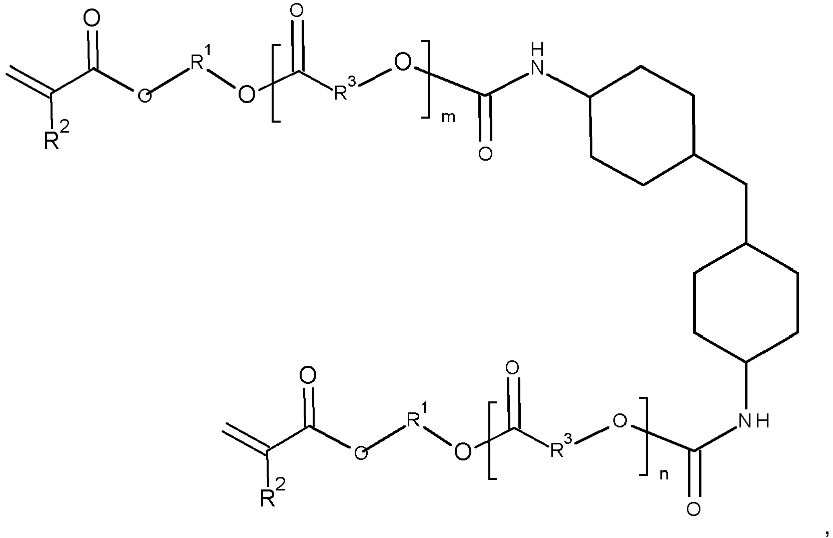 worin R¹ bis R³ die obigen Bedeutungen aufweisen.

Gemäß der vorliegenden Erfindung werden im ersten Schritt Hydroxyalkyl(meth)acrylate (A) der Formel 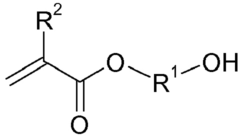 in denen R¹ und R² die oben aufgeführten Bedeutungen hat, mit (n + m)/2 Äquivalenten Lacton (B) der Formel 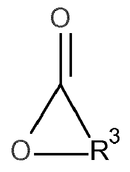 in dem R³ die oben aufgeführten Bedeutungen hat, zu einem Zwischenprodukt der Formel 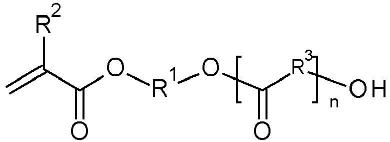 umgesetzt.

Besonders bevorzugt als Hydroxyalkyl(meth)acrylate (A) sind 2-Hydroxyethyl(meth)acrylat, 2- oder 3-Hydroxypropyl(meth)acrylat, 1,4-Butandiolmono(meth)acrylat, Neopentylglykolmono-(meth)acrylat, 1,5-Pentandiolmono(meth)acrylat und 1,6-Hexandiolmono(meth)acrylat, ganz besonders bevorzugt sind 2-Hydroxyethyl(meth)acrylat, 2-Hydroxypropyl(meth)acrylat und 1,4-Butandiolmono(meth)acrylat, insbesondere 2-Hydroxyethyl(meth)acrylat.

Die Acrylate sind dabei jeweils gegenüber den Methacrylaten bevorzugt.

Das Lacton (B) weist folgende Formel auf

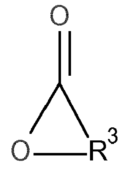

Bevorzugte Lactone sind beta-Propiolacton, gamma-Butyrolacton, gamma-Ethyl-gamma-butyrolacton, gamma-Valerolacton, delta-Valerolacton, epsilon-Caprolacton, 7-Methyloxepan-2-on, 1,4-Dioxepan-5-on, Oxacyclotridecan-2-on und 13-Butyl-oxacyclotridecan-2-on.

Besonders bevorzugt sind gamma-Butyrolacton, delta-Valerolacton und epsilon-Caprolacton, ganz besonders bevorzugt ist epsilon-Caprolacton.

Die Reaktion findet im ersten Schritt erfindungsgemäß in Gegenwart mindestens einer Zink- und/oder Wismutverbindung (C) statt, beispielsweise ein bis drei, bevorzugt ein oder zwei und besonders bevorzugt genau einer Zink- oder Wismutverbindung. Die Zinkverbindungen sind gegenüber den Wismutverbindungen bevorzugt.

Bevorzugte Wismutverbindungen sind solche der Oxidationsstufe +3, bevorzugt Zink verbindungen solche der Oxidationsstufe +2.

Als Zinkverbindungen (C) kommen dabei besonders bevorzugt Zinkverbindungen der Oxidationsstufe +2 in Betracht, mit folgenden Anionen: F⁻, Cl⁻, ClO⁻, ClO3⁻, ClO₄⁻, Br⁻, J⁻, JO3⁻, CN⁻, OCN⁻, NO₂⁻, NO₃⁻, HCO₃⁻, CO₃²⁻, S²⁻, SH⁻, HSO₃⁻, SO₃²⁻, SO₄⁻, SO₄²⁻, S₂O₂²⁻, S₂O₄²⁻, S₂O₅²⁻, S₂O₆²⁻, S₂O₇²⁻, S₂O₈²⁻, H₂PO₂⁻, H₂PO₄⁻, HPO₄²⁻, PO₄³⁻, P₂O₇⁴⁻, (OCₓH₂ₓ₊₁)⁻, (CₓH₂ₓ₋₁O₂)⁻, (CₓH₂ₓ₋₃O₂)⁻ sowie (Cₓ₊₁H₂ₓ₋₂O₄)²⁻, wobei x für die Zahlen 1 bis 20 steht. Bevorzugt sind dabei die Carboxylate, bei denen das Anion den Formeln (CₓH₂ₓ₋₁O₂)⁻ sowie (Cₓ₊₁H₂ₓ₋₂O₄)²⁻ mit n gleich 1 bis 20, gehorcht. Besonders bevorzugte Salze weisen als Anionen Monocarboxylate der allgemeinen Formel (CₓH₂ₓ₋₁O₂)⁻ auf, wobei x für die Zahlen 1 bis 20, bevorzugt 1 bis 10 steht. Hierbei sind insbesondere zu erwähnen Format, Acetat, Propionat, Hexanoat, Neodekanoat und 2-Ethylhexanoat.

Unter den Zink-Katalysatoren sind die Zink-carboxylate bevorzugt, besonders bevorzugt solche von Carboxylaten, die mindestens sechs Kohlenstoffatome aufweisen, insbesondere Zinkoctoate, 2-ethylhexanoate, -neodecanoate, oder -pivalate; beispielsweise Borchi® Kat 22 von OMG Borchers GmbH, Langenfeld, Deutschland.

Als Wismutverbindungen (C) kommen dabei bevorzugt Wismutverbindungen der Oxidationsstufe +3 in Betracht, mit folgenden Anionen: F⁻, Cl⁻, ClO⁻, ClO₃⁻, ClO₄⁻, Br⁻, J⁻, JO₃⁻, CN⁻, OCN⁻, NO₂⁻, NO₃⁻, HCO₃⁻, CO₃²⁻, S²⁻, SH⁻, HSO₃⁻, SO₃²⁻, HSO₄⁻, SO₄²⁻, S₂O₂²⁻, S₂O₄²⁻, S₂O₅²⁻, S₂O₆²⁻, S₂O₇²⁻, S₂O₈²⁻, H₂PO₂⁻, H₂PO₄⁻, HPO₄²⁻, PO₄³⁻, P₂O₇⁴⁻, (OCₓH₂ₓ₊₁)⁻, (CₓH₂ₓ₋₁O₂)⁻, (CₓH₂ₓ₋₃O₂)⁻sowie (Cₓ₊₁H₂ₓ₋₂O₄)²⁻, wobei x für die Zahlen 1 bis 20 steht. Bevorzugt sind dabei die Carboxylate, bei denen das Anion den Formeln (CₓH₂ₓ₋₁O₂)⁻ sowie (Cₓ₊₁H₂ₓ₋₂O₄)²⁻ mit n gleich 1 bis 20, gehorcht. Besonders bevorzugte Salze weisen als Anionen Monocarboxylate der allgemeinen Formel (CₓH₂ₓ₋₁O₂)⁻ auf, wobei x für die Zahlen 1 bis 20, bevorzugt 1 bis 10 steht. Hierbei sind insbesondere zu erwähnen Format, Acetat, Propionat, Hexanoat, Neodekanoat und 2-Ethylhexanoat.

Unter den Wismut-Katalysatoren sind die Wismut-carboxylate bevorzugt, besonders bevorzugt solche von Carboxylaten, die mindestens sechs Kohlenstoffatome aufweisen, insbesondere Wismut-octoate, -ethylhexanoate, -neodecanoate, oder -pivalate; beispielsweise K-KAT 348, XC-B221; XC-C227, XC 8203 und XK-601 von King Industries, TIB KAT 716, 716LA, 716XLA, 718, 720, 789 von TIB Chemicals und solchen von Shepherd Lausanne, sowie beispielsweise Borchi® Kat 24; 315; 320 von OMG Borchers GmbH, Langenfeld, Deutschland.

Es kann sich dabei auch um Gemische verschiedener Metalle handeln, wie beispielsweise in Borchi® Kat 0245 von OMG Borchers GmbH, Langenfeld, Deutschland
Besonders bevorzugt sind jedoch Wismut neodecanoat, Zink neodecanoat, Zink-2-ethylhexanoat und Wismut-2-ethylhexanoat.

Es ist möglich, die Wirkung der Katalysatoren zusätzlich durch Anwesenheit von Säuren zu verstärken, beispielsweise durch Säuren mit einem pKa-Wert von < 2,5, wie beschrieben in EP 2316867 A1 oder mit einem pKa-Wert zwischen 2,8 und 4,5, wie beschrieben in WO 04/029121 A1. Bevorzugt ist die Verwendung von Säuren mit einem pKa-Wert von nicht mehr als 4,8, besonders bevorzugt von nicht mehr als 2,5.

Die Umsetzung der Komponenten (A) und (B) findet bevorzugt statt bei Temperaturen von 50 bis 150 °C, bevorzugt 70 bis 130 °C über einen Zeitraum von 3 bis 48 Stunden, bevorzugt von 5 bis 36 Stunden unter Rühren oder Umpumpen.

Die Komponenten (A) und (B) werden dabei in der gewünschten Stöchiometrie (mol : mol), die bevorzugt 1 : 1,5 bis 3, besonders bevorzugt 1 : 1,8 bis 2,5, ganz besonders bevorzugt 1 : 2 bis 2,3 und insbesonders 1 : 2 beträgt, miteinander vermischt und aufgeheizt. Es kann auch Komponente (A) vorgelegt und (B) erst während oder nach dem Aufheizen zugegeben werden.

Vor, während oder nach dem Aufheizen wird der Katalysator (C), optional verteilt in mehreren Portionen, zu dem Gemisch zugegeben.

Es ist auch möglich, zunächst Komponente (A) mit lediglich einem Teil der Verbindung (B) umzusetzen und den Rest der Verbindung (B) zu einem späteren Zeitpunkt zur Reaktion zuzugeben.

Bevorzugt werden alle drei Komponenten (A), (B) und (C) miteinander vermischt und gemeinsam aufgeheizt und umgesetzt.

Der Katalysator (C) wird in der Regel in Mengen von 0,001 bis 2 Gew% bezogen auf die Summe der Komponenten (A) und (B) dem Reaktionsgemisch zugegeben, bevorzugt 0,005 bis 1,5 Gew%, besonders bevorzugt 0,01 bis 1 und ganz besonders bevorzugt 0,01 bis 0,5 Gew%.

Es ist optional möglich, wenn auch weniger bevorzugt, die Umsetzung in Gegenwart mindestens eines Lösungsmittels durchzuführen.

Beispiele für derartige Lösungsmittel sind aromatische (einschließlich alkylierter Benzole und Naphthaline) und/oder (cyclo)aliphatische Kohlenwasserstoffe und deren Gemische, chlorierte Kohlenwasserstoffe, Ketone, Ester, alkoxylierte Alkansäurealkylester, Ether, respektive Gemische der Lösungsmittel.

Als aromatische Kohlenwasserstoffgemische sind solche bevorzugt, die überwiegend aromatische C7- bis C14-Kohlenwasserstoffe umfassen und einen Siedebereich von 110 bis 300 °C umfassen können, besonders bevorzugt sind Toluol, o-, m- oder p-Xylol, Trimethylbenzolisomere, Tetramethylbenzolisomere, Ethylbenzol, Cumol, Tetrahydronaphthalin und solche enthaltende Gemische.

Beispiele dafür sind die Solvesso®-Marken der Firma ExxonMobil Chemical, besonders Solvesso® 100 (CAS-Nr. 64742-95-6, überwiegend C9 und C10-Aromaten, Siedebereich etwa 154 - 178 °C), 150 (Siedebereich etwa 182 - 207 °C) und 200
(CAS-Nr. 64742-94-5), sowie die Shellsol®-Marken der Firma Shell, Caromax® (z.B. Caromax® 18) der Firma Petrochem Carless und Hydrosol der Firma DHC (z.B. als Hydrosol® A 170). Kohlenwasserstoffgemische aus Paraffinen, Cycloparaffinen und Aromaten sind auch unter den Bezeichnungen Kristallöl (beispielsweise Kristallöl 30, Siedebereich etwa 158 - 198 °C oder Kristallöl 60: CAS-Nr. 64742-82-1), Testbenzin (beispielsweise ebenfalls CAS-Nr. 64742-82-1) oder Solventnaphtha (leicht: Siedebereich etwa 155 - 180 °C, schwer: Siedebereich etwa 225 - 300 °C) im Handel erhältlich. Der Aromatengehalt derartiger Kohlenwasserstoffgemische beträgt in der Regel mehr als 90 Gew%, bevorzugt mehr als 95, besonders bevorzugt mehr als 98 und ganz besonders bevorzugt mehr als 99 Gew%. Es kann sinnvoll sein, Kohlenwasserstoffgemische mit einem besonders verringerten Gehalt an Naphthalin einzusetzen.

(Cyclo)aliphatische Kohlenwasserstoffe sind beispielsweise Dekalin, alkyliertes Dekalin und Isomerengemische von geradlinigen oder verzweigten Alkanen und/oder Cycloalkanen.

Der Gehalt an aliphatischen Kohlenwasserstoffen beträgt in der Regel weniger als 5, bevorzugt weniger als 2,5 und besonders bevorzugt weniger als 1 Gew%.

Ester sind beispielsweise n-Butylacetat, Ethylacetat, 1-Methoxypropylacetat-2 und 2-Methoxyethylacetat.

Ether sind beispielsweise THF, Dioxan sowie die Dimethyl-, -ethyl- oder -n-butylether von Ethylenglykol, Diethylenglykol, Triethylenglykol, Propylenglykol, Dipropylenglykol oder Tripropylenglykol.

Ketone sind beispielsweise Aceton, Diethylketon, Ethylmethylketon, Isobutylmethylketon, Methylamylketon und tert.-Butylmethylketon.

Bevorzugte Lösungsmittel sind n-Butylacetat, Ethylacetat, 1-Methoxypropylacetat-2, 2-Methoxyethylacetat, sowie deren Gemische, insbesondere mit den oben aufgeführten aromatischen Kohlenwasserstoffgemischen, insbesondere Xylol und Solvesso® 100.

Derartige Gemische können im Volumenverhältnis 5:1 bis 1:5 erstellt werden, bevorzugt im Volumenverhältnis 4:1 bis 1:4, besonders bevorzugt im Volumenverhältnis 3:1 bis 1:3 und ganz besonders bevorzugt im Volumenverhältnis 2:1 bis 1:2.

Bevorzugte Beispiele sind Butylacetat/Xylol, Methoxypropylacetat/Xylol 1:1, Butylacetat/Solventnaphtha 100 1:1, Butylacetat/Solvesso® 100 1:2 und Kristallöl 30/Shellsol® A 3:1. Bevorzugt sind Butylacetat, 1-Methoxypropylacetat-2, Methylamylketon, Xylol und Solvesso® 100.

In der Regel ist es erforderlich und bevorzugt, die Reaktion in Gegenwart mindestens eines Stabilisators gegen radikalische Polymerisation der Komponente (A) durchzuführen, bevorzugt Hydrochinonmonomethylether und/oder Phenothizin. Es ist aber auch möglich andere Stabilisatoren einzusetzen, die für die Stabilisierung von (Meth)acrylaten gegen radikalische Polymerisation bekannt sind.

Der erste Reaktionsschritt ist dann beendet, wenn das Lacton (B) im wesentlichen umgesetzt ist, bevorzugt zu mindestens 90%, besonders bevorzugt zu mindestens 95, ganz besonders bevorzugt zu mindestens 97 und insbesondere zu mindestens 98%.

Es ist möglich, umumgesetztes Lacton (B) sowie optional eingesetztes Lösungsmittel aus dem Reaktionsgemisch zu entfernen, bevorzugt per Destillation, es stellt jedoch eine bevorzugte Ausführungsform dar, das aus dem ersten Schritt erhaltene Reaktionsgemisch direkt in den zweiten Schritt, die Umsetzung mit Komponente (D), einzusetzen.

Es ist möglich, die Reaktion aus dem ersten Schritt bevorzugt durch Abkühlen abzubrechen. Das Reaktionsgemisch ist in dieser Form lagerfähig und kann dann zu einem späteren Zeitpunkt in den zweiten Schritt eingesetzt werden.

Im zweiten Reaktionsschritt wird das aus dem ersten Schritt erhaltene Reaktionsgemisch dann mit Komponente (D) umgesetzt.

Bei Komponente (D) handelt es sich um mindestens ein, bevorzugt genau ein cycloaliphatisches oder unsymmetrisches aliphatisches Diisocyanat.

Cycloaliphatische Diisocyanate sind solche Diisocyanate, bei denen mindestens eine Isocyanatgruppe an ein cyclisches, nichtaromatisches Ringsystem gebunden ist.

Aliphatische Diisocyanate sind solche, bei denen beide Isocyanatgruppen jeweils an ein sp³-hybridisiertes Kohlenstoffatom gebunden sind, das nicht Bestandteil eines Ringsystems ist.

Unsymmetrische aliphatische Diisocyanate sind solche aliphatischen Diisocyanate, in denen die Isocyanatgruppen mit einem zweibindigen organischen Rest verbunden sind, der keine Spiegelebene aufweist, die senkrecht auf die Achse steht, mit der die beiden Isocyanatgruppen verbunden sind.

Bevorzugte cycloaliphatische Diisocyanate sind 1,4-, 1,3- oder 1,2-Diisocyanatocyclohexan, 4,4'- oder 2,4'-Di(isocyanatocyclohexyl)methan, Bis(isocyanatomethyl)-bicyclo[2.2.1]heptan (NBDI), 1-Isocyanato-3,3,5-trimethyl-5-(isocyanatomethyl)cyclohexan (Isophorondiisocyanat), 1,3- oder 1,4-Bis(isocyanatomethyl)cyclohexan oder 2,4-, oder 2,6-Diisocyanato-1-methylcyclohexan sowie 3 (bzw. 4), 8 (bzw. 9)-Bis(isocyanatomethyl)-tricyclo[5.2.1.0^{2.6}]decan-Isomerengemische.

Bevorzugte unsymmetrische aliphatische Diisocyanate sind Derivate des Lysindiisocyanates, Tetramethylxylylendiisocyanat, Trimethylhexandiisocyanat oder Tetramethylhexandiisocyanat.

Besonders bevorzugte Verbindungen (D) sind Isophorondiisocyanat, 4,4'- Di(isocyanatocyclohexyl)methan, 2,2,4- und 2,4,4-Trimethylhexandiisocyanat, ganz besonders bevorzugt sind Di(isocyanatocyclohexyl)methan, 2,2,4- und 2,4,4-Trimethylhexandiisocyanat und insbesondere 4,4'-Di(isocyanatocyclohexyl)methan.

Dicyclohexylmethan-4,4'-diisocyanat kann als Gemisch der verschiedenen cis- und trans-Isomere vorliegen und kann auch einen Anteil an 2,4'-Di(isocyanatocyclohexyl)methan enthalten.

Für die vorliegende Erfindung können sowohl solche Diisocyanate eingesetzt werden, die durch Phosgenierung der korrespondierenden Amine erhalten werden, als auch solche, die ohne die Verwendung von Phosgen, d. h. nach phosgenfreien Verfahren, hergestellt werden. So erhaltene Diisocyanate weisen in der Regel einen sehr geringen oder sogar nicht messbaren Anteil an chlorierten Verbindungen auf, was beispielsweise in Anwendungen in der Elektronikindustrie vorteilhaft ist.

In einer Ausführungsform der vorliegenden Erfindung weisen die eingesetzten Isocyanate an hydrolysierbarem Chlor weniger als 100 ppm auf, bevorzugt weniger als 50 ppm, insbesondere weniger als 30 ppm und speziell weniger als 20 ppm. Dies kann beispielsweise gemessen werden durch die ASTM-Vorschrift D4663-98. Die Gehalte an gesamtem Chlor liegen beispielsweise bei unter 1000 ppm, bevorzugt unter 800 ppm und besonders bevorzugt unter 500 ppm (ermittelt per argentometrischer Titration nach Hydrolyse).

Der zweite Reaktionsschritt wird in einer Stöchiometrie von 1,2: 1 bis 1:1,2 von Hydroxygruppen im Reaktionsprodukt aus dem ersten Schritt zu Isocyanatgruppen in Komponente (D) durchgeführt, bevorzugt 1,1 : 1 bis 1 : 1,1, besonders bevorzugt 1,05 : 1 bis 1: 1,05 und ganz besonders bevorzugt 1:1.

Die Umsetzung im zweiten Schritt erfolgt bevorzugt bei 40 bis 100 °C, besonders bevorzugt 50 bis 90, ganz besonders bevorzugt bei 60 bis 80 °C.

Dazu wird das aus dem ersten Reaktionsschritt erhaltene Reaktionsgemisch auf die gewünschte Temperatur gebracht und die Komponente (D) in mehreren oder bevorzugt in einer Portion eingetragen.

In der Regel und bevorzugt ist der aus der Reaktion im ersten Schritt im Reaktionsgemisch befindliche Katalysator (C) ausreichend, um auch die Reaktion zwischen Isocyanat- und Hydroxygruppen zu katalysieren. Sollte dies nicht der Fall sein, so kann noch weiterer Katalysator (C) nachdosiert werden.

Die Reaktion wird weitergeführt, bis der NCO-Wert auf unter 1 Gew%, bevorzugt unter 0,5 Gew%, besonders bevorzugt unter 0,3, ganz besonders bevorzugt unter 0,1 und insbesondere unter 0,1 Gew% gesunken ist.

Sollte die Reaktion in Gegenwart eines Lösungsmittels durchgeführt worden sein, so kann dieses jetzt abgetrennt werden, bevorzugt destillativ.

Es ist möglich, wenn auch in der Regel nicht erforderlich, den Katalysator aus dem erhaltenen Reaktionsgemisch abzutrennen.

Dies kann beispielsweise durch eine Wäsche oder Filtration erfolgen.

Dazu wird das Reaktionsgemisch in einem Waschapparat mit einer 5 - 25, bevorzugt 5 - 20, besonders bevorzugt 5 - 15 Gew%igen wäßrigen Lösung einer Base, wie z.B. Natronlauge, Kalilauge, Natriumhydrogencarbonat, Natriumcarbonat, Kaliumhydrogencarbonat, Kalziumhydroxid, Ammoniakwasser oder Kaliumcarbonat, der gegebenenfalls 5 - 15 Gew% Kochsalz, Kaliumchlorid, Ammoniumchlorid oder Ammoniumsulfat zugesetzt sein können, bevorzugt mit Natronlauge oder Natronlauge-Kochsalz-Lösung, neutralisiert.

Die Wäsche kann beispielsweise in einem Rührbehälter oder in anderen herkömmlichen Apparaturen, z.B. in einer Kolonne oder Mixer-Settler-Apparatur, durchgeführt werden.

Anschließend wird die organische Phase der Vorwäsche mit Wasser oder einer 5 - 30 Gew%igen, bevorzugt 5 - 20, besonders bevorzugt 5 - 15 Gew%-igen Kochsalz-, Kaliumchlorid-, Ammoniumchlorid-, Natriumsulfat- oder Ammoniumsulfatlösung, bevorzugt Kochsalzlösung, behandelt.

Es ist jedoch auch möglich, Katalysatorspuren aus dem Reaktionsgemisch durch dessen Filtration über Aktivkohle, Aluminiumoxid, Silika oder Ionentauscher zu entfernen.

Das gemäß dem erfindungsgemäßen Verfahren erhaltene Produkt kann in an sich bekannter Weise in strahlungshärtbare Beschichtungsmassen eingesetzt werden und weist als einen Vorteil auf, daß im Produkt der ersten Stufe die Verteilung der Lactoneinheiten (B) gleichmäßiger ist als gemäß den Verfahren aus dem Stand der Technik. Dies hat zur Folge, daß die Beschichtungsmassen, die ein Produkt erhalten nach dem erfindungsgemäßen Verfahren enthalten, eine hohe Flexibilität aufweisen.

Dementsprechend ist auch die Verwendung von Urethan(meth)acrylaten, erhalten nach dem erfindungsgemäßen Verfahren, in strahlungshärtbaren Beschichtungsmassen ein Gegenstand der vorliegenden Erfindung.

Die derartigen Beschichtungsmassen eigenen sich zur Beschichtung von Substraten wie Holz, Papier, Textil, Leder, Vlies, Kunststoffoberflächen, PVC, Glas, Keramik, mineralischen Baustoffen, wie Zement-Formsteinen und Faserzementplatten, oder Metallen oder beschichteten Metallen, bevorzugt von Kunststoffen oder Metallen, insbesondere in Form von Folien, besonders bevorzugt Metallen.

Die Beschichtungsmittel können insbesondere in Grundierungen, Füllern, pigmentierten Decklacken und Klarlacken im Bereich Autoreparatur- oder Großfahrzeuglackierung und Flugzeugen eingesetzt werden. Besonders geeignet sind solche Beschichtungsmittel für Anwendungen, in denen eine besonders hohe Applikationssicherheit, Außenwitterungsbeständigkeit, Härte und Flexibilität gefordert werden, wie in der Autoreparatur- und Großfahrzeuglackierung.

Die im Folgenden angegebenen Beispiele sollen die vorliegende Erfindung erläutern, ohne sie jedoch einzuschränken.

Die in dieser Schrift angegebenen %- und ppm-Angaben beziehen sich auf Gew.% und Gew.ppm, soweit nicht anders angegeben.

### Beispiele

### Beispiel 1:

323 Teile epsilon-Caprolacton, 164 Teile Hydroxyethylacrylat und 0,5 Teile Wismut 2-ethylhexanoat (BorchiKat® 24 der OMG Borchers GmbH, Langenfeld, Deutschland) wurden 35 Stunden bei 105 - 110°C erhitzt, dann wurde auf 60°C abgekühlt und 187 Teile eines Diisocyanates auf Basis H12-MDI (Desmodur® W der Bayer MaterialScience) zugegeben und weitere 14 Stunden bei 80-85°C reagieren lassen. Der Isocyanatwert war auf < 0,1% abgefallen. Es entstand ein zähflüssiges, klares Urethanacrylat mit einer Viskosität von 27,5 Pas (gemessen mit einem Epprecht Kegel/Platte Viskosimeter (Cone C) bei 23 °C. Die GPC Chromatogramme der ersten und zweiten Stufe zeigen etwas weniger niedermolekulare Produkte als die des Vergleichsbeispiels 1, in dem die erste Stufe mit einem zinnorganischen Katalysator hergestellt wurde.

### Beispiel 2:

323 Teile epsilon-Caprolacton, 164 Teile Hydroxyethylacrylat und 0,1 Teile Zink 2-ethylhexanoat (BorchiKat® 22 der OMG Borchers GmbH, Langenfeld, Deutschland) wurden 11 Stunden bei 105 - 110°C erhitzt, dann wurde auf 60°C abgekühlt und 187 Teile eines Diisocyanates auf Basis H12-MDI (Desmodur® W der Bayer MaterialScience) zugegeben und weitere 14 Stunden bei 80-85°C reagieren lassen. Der Isocyanatwert war auf < 0,1 % abgefallen. Es entstand ein zähflüssiges, klares Urethanacrylat mit einer Viskosität von 24,0 Pas (gemessen mit einem Epprecht Kegel/Platte Viskosimeter (Cone C) bei 23 °C. Die GPC Chromatogramme der ersten und zweiten Stufe entsprechen denen des Beispiels 1.

### Vergleichsbeispiel 1:

323 Teile epsilon-Caprolacton, 164 Teile Hydroxyethylacrylat und 0,05 Butyl-Zinn-tris(2-ethylhexanoat) wurden 11 Stunden bei 105 - 110°C erhitzt, dann wurde auf 60°C abgekühlt und 187 Teile eines Diisocyanates auf Basis H12-MDI (Desmodur® W der Bayer MaterialScience) zugegeben und weitere 14 Stunden bei 80-85°C reagieren lassen. Der Isocyanatwert war auf < 0,1% abgefallen. Es entstand ein zähflüssiges, klares Urethanacrylat mit einer Viskosität von 19,8 Pas (gemessen mit einem Epprecht Kegel/Platte Viskosimeter (Cone C).

### Vergleichsbeispiele der ersten Stufe

323 Teile epsilon-Caprolacton, 164 Teile Hydroxyethylacrylat und x Teile Katalysator wie in der Tabelle angegeben wurden y Stunden wie in der Tabelle angegeben bei 105 - 110°C erhitzt. Anschließend wurde die Viskosität des erhaltenen Reaktionsgemisches bei 23 °C bestimmt.

| Katalysator | Katalysatormenge/Reaktionszeit | Viskosität (mPas) |
|---|---|---|
| Butyl-zinn-tris ethylhexanoat | 0,01/11 | 85 |
| Wismut-ethylhexanoat (erfindungsgemäß) | 0,2/35 | 95 |
| Tetrabutyl-o-titanat | 0,1/11 | 55 |
| Phosphorsäure | 0,05/11 | 100 |
| p-Toluolsulfonsäure | 0,1/11 | 125 |
| Zink ethylhexanoat (erfindungsgemäß) | 0,1/11 | 80 |
| Cäsium acetat | 0,1/11 | 5 |

Die GPC-Daten (gemessen durch Gelpermeationschromatographie mit Tetrahydrofuran und Polystyrol als Standard) der ersten Stufe mit folgenden Katalysatoren:

| | |
|---|---|
| p-Toluolsulfonsäure | Mn = 546 g/mol |
| Butyl-zinn tris-ethylhexanoat | Mn = 460 g/mol |
| Zink ethylhexanoat | Mn = 455 g/mol |
| Tetra butyl-titanat | Mn = 401 g/mol |

Man sieht anhand der Viskosität und des zahlenmittleren Molgewichts Mn, daß die Brönsted-Katalysatoren Phosphorsäure und p-Toluolsulfonsäure im Vergleich zur Zinnkatalyse ein höhermolekulares Produkt liefern.

Katalyse mit Tetrabutyl-o-titanat oder Cäsium acetat liefert dagegen ein Produkt, welches das Molekulargewicht des Produktes der Zinnkatalyse nicht erreicht.

Hingegen wird mit der erfindungsgemäßen Zink- und Wismutkatalyse Viskosität und Produktspektrum des Vergleichsproduktes erreicht.

### Beispiel 3: Bestimmung der Flexibilität

60 Teile der jeweiligen Oligomere aus den Beispielen 1 und 2, sowie aus dem Vergleichsbeispiel wurden mit 40 Teilen Dipropylenglykoldiacrylat, 4,5 Teilen des Photoinitiators Benzophenon und 4 Teilen des Photoinitiators Darocure® 1173 abgemischt und auf eine starre Polyvinylchloridfolie in einer Schichtdicke von ca. 12 µm aufgebracht. Dann wurde auf einer IST Bandanlage mit einer Geschwindigkeit von 10 m/min (ca. 1400 mJ/cm²) mit UV Strahlung belichtet.

Die so mit einer Lackschicht überzogenen Folien wurden einem Dornbiegetest unterzogen, indem die Folie um einen Dorn mit abnehmendem Durchmesser gezogen wurde und beurteilt wurde, ob sich in der Lackschicht Risse zeigen. Je kleiner der Durchmesser des Dorns wird, desto höher muß die Flexibilität der Lackschicht sein.

| Nr | 15 mm Dorndurchmesser | 10 mm Dorndurchmesser |
|---|---|---|
| Beispiel 1 | ok | ok |
| Beispiel 2 | ok | ok |
| Vergleichsbeispiel 1 | ok | Risse |

## Patentansprüche

1. Verfahren zur Herstellung von Urethan(meth)acrylaten der Formel 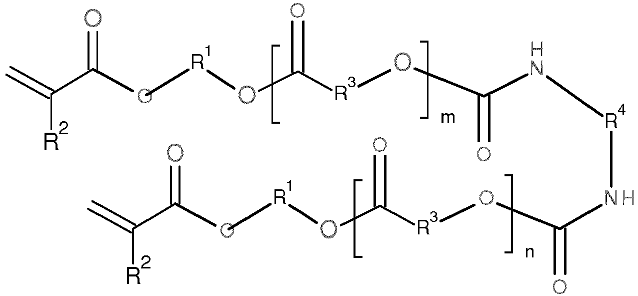 **dadurch gekennzeichnet, daß** man in einem ersten Schritt ein Hydroxyalkyl(meth)acrylat (A) der Formel 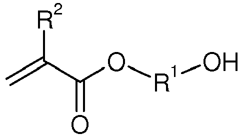 mit einem Lacton (B) der Formel 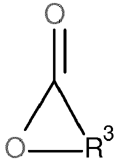 in Gegenwart mindestens einer Zink- und/oder Wismutverbindung (C) miteinander umsetzt,
und in einem weiteren Schritt das so erhaltene zink- bzw. bismuthaltige Produkt aus dem ersten Schritt mit mindestens einem cycloaliphatischen oder unsymmetrischen aliphatischen Diisocyanat (D) umsetzt,
worin R¹ einen zweiwertigen, 2 bis 12 Kohlenstoffatome aufweisenden Alkylenrest, der gegebenenfalls mit C₁- bis C₄-Alkylgruppen substituiert und/oder durch ein oder mehrere Sauerstoffatome unterbrochen sein kann,
R² jeweils unabhängig voneinander Methyl oder Wasserstoff,
R³ einen zweiwertigen, 1 bis 12 Kohlenstoffatome aufweisenden Alkylenrest, der gegebenenfalls mit C₁- bis C₄-Alkylgruppen und/oder durch ein oder mehrere Sauerstoffatome unterbrochen substituiert sein kann,
R⁴ einen zweiwertigen organischen Rest darstellt, der durch Abstraktion beider Isocyanatgruppen aus einem cycloaliphatischen oder unsymmetrischen aliphatischen Diisocyanat entsteht, und
n und m unabhängig voneinander positive Zahlen von 1 bis 5,
bedeuten.

2. Verfahren gemäß Anspruch 1, **dadurch gekennzeichnet, daß** R¹ ausgewählt ist aus der Gruppe bestehend aus 1,2-Ethylen, 1,2- oder 1,3-Propylen, 1,2-, 1,3- oder 1,4-Butylen, 1,1-Dimethyl-1,2-ethylen, 1,2-Dimethyl-1,2-ethylen, 1,5-Pentylen, 1,6-Hexylen, 1,8-Octylen, 1,10-Decylen und 1,12-Dodecylen.

3. Verfahren gemäß Anspruch 1 oder 2, **dadurch gekennzeichnet, daß** R³ ausgewählt ist aus der Gruppe bestehend aus Methylen, 1,2-Ethylen, 1,2-Propylen, 1,3-Propylen, 1,2-Butylen, 1,3-Butylen, 1,4-Butylen, 1,5-Pentylen, 1,5-Hexylen, 1,6-Hexylen, 1,8-Octylen, 1,10-Decylen, 1,12-Dodecylen, 2-Oxa-1,4-butylen, 3-Oxa-1,5-pentylen und 3-Oxa-1,5-hexylen.

4. Verfahren gemäß einem der vorstehenden Ansprüche, **dadurch gekennzeichnet, daß** (A) ausgewählt ist aus der Gruppe bestehend aus 2-Hydroxyethyl(meth)acrylat, 2- oder 3-Hydroxypropyl(meth)acrylat, 1,4-Butandiolmono(meth)acrylat, Neopentylglykolmono(meth)acrylat, 1,5-Pentandiolmono(meth)acrylat und 1,6-Hexandiolmono(meth)acrylat.

5. Verfahren gemäß einem der vorstehenden Ansprüche, **dadurch gekennzeichnet, daß** (B) ausgewählt ist aus der Gruppe bestehend aus beta-Propiolacton, gamma-Butyrolacton, gamma-Ethyl-gamma-butyrolacton, gamma-Valerolacton, delta-Valerolacton, epsilon-Caprolacton, 7-Methyloxepan-2-on, 1,4-Dioxepan-5-on, Oxacyclotridecan-2-on und 13-Butyl-oxacyclotridecan-2-on.

6. Verfahren gemäß einem der vorstehenden Ansprüche, **dadurch gekennzeichnet, daß** es sich bei der Wismutverbindung (C) um eine Wismutverbindung der Oxidationsstufe +3 handelt mit einem Anion ausgewählt aus der Gruppe bestehend aus F⁻, Cl⁻, ClO⁻, ClO₃⁻, ClO₄⁻, Br⁻, J⁻, JO₃⁻, CN⁻, OCN⁻, NO₂⁻, NO₃⁻, HCO₃⁻, CO₃²⁻, S²⁻, SH⁻, HSO₃⁻, SO₃²⁻, HSO₄⁻, SO₄²⁻, S₂O₂²⁻, S₂O₄²⁻, S₂O₅²⁻, S₂O₆²⁻, S₂O₇²⁻, S₂O₈²⁻, H₂PO₂⁻, H₂PO₄⁻, HPO₄²⁻, PO₄³⁻, P₂O₇⁴⁻, (OCₓH₂ₓ₊₁)⁻, (CₓH₂ₓ₋₁O₂)⁻, (CₓH₂ₓ₋₃O₂)⁻ sowie (Cₓ₊₁H₂ₓ₋₂O₄)²⁻ handelt, wobei x für die Zahlen 1 bis 20 steht.

7. Verfahren gemäß einem der Ansprüche 1 bis 5, **dadurch gekennzeichnet, daß** es sich bei der Wismutverbindung (C) um ein Wismut carboxylat handelt.

8. Verfahren gemäß einem der Ansprüche 1 bis 5, **dadurch gekennzeichnet, daß** es sich bei der Wismutverbindung (C) um Wismut Format, Acetat, Propionat, Hexanoat, Neodekanoat, 2-Ethylhexanoat, Octoate, oder Pivalat handelt.

9. Verfahren gemäß einem der Ansprüche 1 bis 5, **dadurch gekennzeichnet, daß** es sich bei der Zinkverbindung (C) um solche der Oxidationsstufe +2 handelt, mit einem Anion ausgewählt aus der Gruppe bestehend aus F⁻, Cl⁻, ClO⁻, ClO₃⁻, ClO₄⁻, Br⁻, J⁻, JO₃⁻, CN⁻, OCN⁻, NO₂⁻, NO₃⁻, HCO₃⁻, CO₃²⁻, S²⁻, SH⁻, HSO₃⁻, SO₃²⁻, HSO₄⁻, SO₄²⁻, S₂O₂²⁻, S₂O₄²⁻, S₂O₅²⁻, S₂O₆²⁻, S₂O₇²⁻, S₂O₈²⁻, H₂PO₂⁻, H₂PO₄⁻, HPO₄²⁻, PO₄³⁻, P₂O₇⁴⁻, (OCₓH₂ₓ₊₁)⁻, (CₓH₂ₓ₋₁O₂)⁻, (CₓH₂ₓ₋₃O₂)⁻ sowie (Cₓ₊₁H₂ₓ₋₂O₄)²⁻, wobei x für die Zahlen 1 bis 20 steht.

10. Verfahren gemäß einem der vorstehenden Ansprüche, **dadurch gekennzeichnet, daß** man den ersten Schritt bei Temperaturen von 50 bis 150 °C über einen Zeitraum von 3 bis 48 Stunden durchführt.

11. Verfahren gemäß einem der vorstehenden Ansprüche, **dadurch gekennzeichnet, daß** im ersten Schritt die Stöchiometrie der Komponenten (A) und (B) 1 : 1,5 bis 3 beträgt.

12. Verfahren gemäß einem der vorstehenden Ansprüche, **dadurch gekennzeichnet, daß** man den Katalysator (C) in Mengen von 0,001 bis 2 Gew% bezogen auf die Summe der Komponenten (A) und (B) dem Reaktionsgemisch zugibt.

13. Verfahren gemäß einem der vorstehenden Ansprüche, **dadurch gekennzeichnet, daß** man den zweiten Schritt bei 40 bis 100 °C durchführt.

14. Verfahren gemäß einem der vorstehenden Ansprüche, **dadurch gekennzeichnet, daß** man den zweiten Schritt bei einer Stöchiometrie von 1,2: 1 bis 1:1,2 von Hydroxygruppen im Reaktionsprodukt aus dem ersten Schritt zu Isocyanatgruppen in Komponente (D) durchfü h rt.

15. Verwendung von Urethan(meth)acrylaten, erhalten nach dem Verfahren gemäß einem der vorstehenden Patentansprüche, in strahlungshärtbaren Beschichtungsmassen.

## Claims

1. A process for preparing urethane (meth)acrylates of the formula 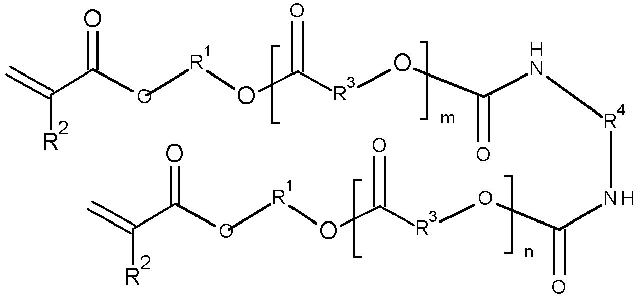 which comprises in a first step reacting a hydroxyalkyl (meth)acrylate (A) of the formula 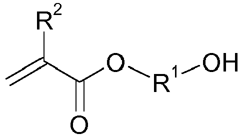 with a lactone (B) of the formula 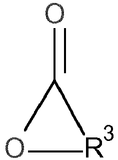 in the presence of at least one zinc compound and/or bismuth compound (C),
and in a further step reacting the resultant bismuth-containing product from the first step with at least one cycloaliphatic or asymmetric aliphatic diisocyanate (D),
in which R¹ is a divalent alkylene radical which has 2 to 12 carbon atoms and which may optionally be substituted by C₁ to C₄ alkyl groups and/or interrupted by one or more oxygen atoms,
R² in each case independently of any other is methyl or hydrogen,
R³ is a divalent alkylene radical which has 1 to 12 carbon atoms and which may optionally be substituted interrupted by C₁ to C₄ alkyl groups and/or by one or more oxygen atoms,
R⁴ is a divalent organic radical which is formed by abstraction of both isocyanate groups from a cycloaliphatic or asymmetric aliphatic diisocyanate, and
n and m independently of one another are positive numbers from 1 to 5.

2. A process according to claim 1, wherein R¹ is selected from the group consisting of 1,2-ethylene, 1,2- or 1,3-propylene, 1,2-, 1,3-, or 1,4-butylene, 1,1-dimethyl-1,2-ethylene, 1,2-dimethyl-1,2-ethylene, 1,5-pentylene, 1,6-hexylene, 1,8-octylene, 1,10-decylene, and 1,12-dodecylene.

3. A process according to claim 1 or 2, wherein R³ is selected from the group consisting of methylene, 1,2-ethylene, 1,2-propylene, 1,3-propylene, 1,2-butylene, 1,3-butylene, 1,4-butylene, 1,5-pentylene, 1,5-hexylene, 1,6-hexylene, 1,8-octylene, 1,10-decylene, 1,12-dodecylene, 2-oxa-1,4-butylene, 3-oxa-1,5-pentylene, and 3-oxa-1,5-hexylene.

4. The process according to any of the preceding claims, wherein (A) is selected from the group consisting of 2-hydroxyethyl (meth)acrylate, 2- or 3-hydroxypropyl (meth)acrylate, 1,4-butanediol mono(meth)acrylate, neopentyl glycol mono(meth)acrylate, 1,5-pentanediol mono(meth)acrylate, and 1,6-hexanediol mono(meth)acrylate.

5. The process according to any of the preceding claims, wherein (B) is selected from the group consisting of beta-propiolactone, gamma-butyrolactone, gamma-ethyl-gamma-butyrolactone, gamma-valerolactone, delta-valerolactone, epsilon-caprolactone, 7-methyloxepan-2-one, 1,4-dioxepan-5-one, oxacyclotridecan-2-one, and 13-butyl-oxacyclotridecan-2-one.

6. The process according to any of the preceding claims, wherein the bismuth compound (C) is a compound of bismuth in the +3 oxidation state with an anion selected from the group consisting of F⁻, Cl⁻, ClO⁻, ClO₃⁻, ClO₄⁻, Br⁻, I⁻, IO₃⁻, CN⁻, OCN⁻, NO₂⁻, NO₃⁻, HCO₃⁻, CO₃²⁻, S²⁻, SH⁻, HSO₃⁻, SO₃²⁻, HSO₄⁻, SO₄²⁻, S₂O₂²⁻, S₂O₄²⁻, S₂O₅²⁻, S₂O₆²⁻, S₂O₇²⁻, S₂O₈²⁻, H₂PO₂⁻, H₂PO₄⁻, HPO₄²⁻, PO₄³⁻, P₂O₇⁴⁻, (OCₓH₂ₓ₊₁)⁻, (CₓH₂ₓ₋₁O₂)⁻, (CₓH₂ₓ₋₃O₂)⁻, and (Cₓ₊₁H₂ₓ₋₂O₄)²⁻, where x stands for the numbers 1 to 20.

7. The process according to any of claims 1 to 5, wherein the bismuth compound (C) is a bismuth carboxylate.

8. The process according to any of claims 1 to 5, wherein the bismuth compound (C) is bismuth formate, acetate, propionate, hexanoate, neodecanoate, 2-ethylhexanoate, octoates, or pivalate.

9. The process according to any of claims 1 to 5, wherein the zinc compound (C) is a compound of zinc in the +2 oxidation state with an anion selected from the group consisting of F⁻, Cl⁻, ClO⁻, ClO₃⁻, ClO₄⁻, Br⁻, I⁻, IO₃⁻, CN⁻, OCN⁻, NO₂⁻, NO₃⁻, HCO₃⁻, CO₃²⁻, S²⁻, SH⁻, HSO₃⁻, SO₃²⁻, HSO₄⁻, SO₄²⁻, S₂O₂²⁻, S₂O₄²⁻, S₂O₅²⁻, S₂O₆²⁻, S₂O₇²⁻, S₂O₈²⁻, H₂PO₂⁻, H₂PO₄⁻, HPO₄²⁻, PO₄³⁻, P₂O₇⁴⁻, (OCₓH₂ₓ₊₁)⁻, (CₓH₂ₓ₋₁O₂)⁻, (CₓH₂ₓ₋₃O₂)⁻, and (Cₓ₊₁H₂ₓ₋₂O₄)²⁻, where x stands for the numbers 1 to 20.

10. The process according to any of the preceding claims, wherein the first step is carried out at temperatures from 50 to 150°C over a period from 3 to 48 hours.

11. The process according to any of the preceding claims, wherein the stoichiometry of components (A) and (B) in the first step is 1:1.5 to 3.

12. The process according to any of the preceding claims, wherein the catalyst (C) is added to the reaction mixture in amounts from 0.001 to 2 wt%, based on the sum of components (A) and (B).

13. The process according to any of the preceding claims, wherein the second step is carried out at 40 to 100°C.

14. The process according to any of the preceding claims, wherein the second step is carried out at a stoichiometry from 1.2:1 to 1:1.2 of hydroxyl groups in the reaction product from the first step to isocyanate groups in component (D).

15. The use of urethane (meth)acrylates obtained by the process according to any of the preceding claims in radiation-curable coating materials.

## Revendications

1. Procédé de fabrication de (méth)acrylates d'uréthane de formule 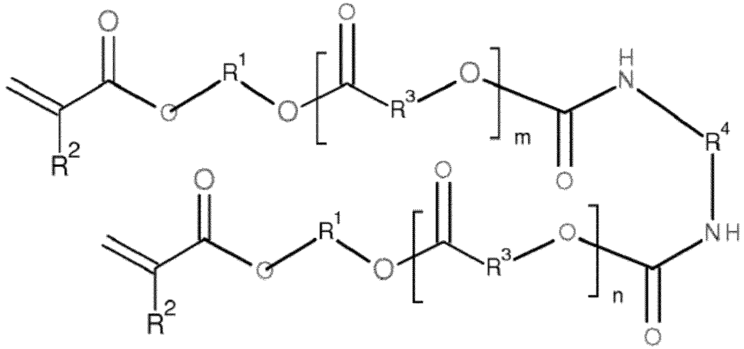 **caractérisé en ce que**, lors d'une première étape, un (méth)acrylate d'hydroxyalkyle (A) de formule 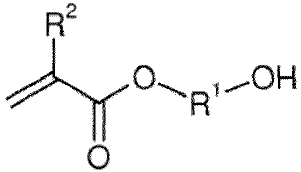 est mis en réaction avec une lactone (B) de formule 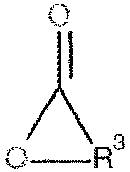 en présence d'au moins un composé de zinc et/ou de bismuth (C),
et lors d'une étape supplémentaire, le produit contenant du zinc ou du bismuth ainsi obtenu à la première étape est mis en réaction avec au moins un diisocyanate cycloaliphatique ou aliphatique asymétrique (D),
R¹ signifiant un radical alkylène bivalent comprenant 2 à 12 atomes de carbone, qui peut éventuellement être substitué avec des groupes alkyle en C₁ à C₄ et/ou interrompu par un ou plusieurs atomes d'oxygène,
les R² signifiant chacun indépendamment les uns des autres méthyle ou hydrogène,
R³ signifiant un radical alkylène bivalent comprenant 1 à 12 atomes de carbone, qui peut éventuellement être substitué avec interrompu des groupes alkyle en C₁ à C₄ et/ou par un ou plusieurs atomes d'oxygène,
R⁴ signifiant un radical organique bivalent, qui est formé par abstraction des deux groupes isocyanate d'un diisocyanate cycloaliphatique ou aliphatique asymétrique, et
n et m signifiant indépendamment l'un de l'autre des nombres positifs de 1 à 5.

2. Procédé selon la revendication 1, **caractérisé en ce que** R¹ est choisi dans le groupe constitué par 1,2-éthylène, 1,2- ou 1,3-propylène, 1,2-, 1,3- ou 1,4-butylène, 1,1-diméthyl-1,2-éthylène, 1,2-diméthyl-1,2-éthylène, 1,5-pentylène, 1,6-hexylène, 1,8-octylène, 1,10-décylène et 1,12-dodécylène.

3. Procédé selon la revendication 1 ou 2, **caractérisé en ce que** R³ est choisi dans le groupe constitué par méthylène, 1,2-éthylène, 1,2-propylène, 1,3-propylène, 1,2-butylène, 1,3-butylène, 1,4-butylène, 1,5-pentylène, 1,5-hexylène, 1,6-hexylène, 1,8-octylène, 1,10-décylène, 1,12-dodécylène, 2-oxa-1,4-butylène, 3-oxa-1,5-pentylène et 3-oxa-1,5-hexylène.

4. Procédé selon l'une quelconque des revendications précédentes, **caractérisé en ce que** (A) est choisi dans le groupe constitué par le (méth)acrylate de 2-hydroxyéthyle, le (méth)acrylate de 2- ou 3-hydroxypropyle, le mono(méth)acrylate de 1,4-butanediol, le mono(méth)acrylate de néopentylglycol, le mono(méth)acrylate de 1,5-pentanediol et le mono(méth)acrylate de 1,6-hexanediol.

5. Procédé selon l'une quelconque des revendications précédentes, **caractérisé en ce que** (B) est choisi dans le groupe constitué par la bêta-propiolactone, la gamma-butyrolactone, la gamma-éthyl-gamma-butyrolactone, la gamma-valérolactone, la delta-valérolactone, l'epsilon-caprolactone, la 7-méthyloxépan-2-one, la 1,4-dioxépan-5-one, l'oxacyclotridécan-2-one et la 13-butyl-oxacyclotridécan-2-one.

6. Procédé selon l'une quelconque des revendications précédentes, **caractérisé en ce que** le composé de bismuth (C) est un composé de bismuth de niveau d'oxydation +3 avec un anion choisi dans le groupe constitué par F⁻, Cl⁻, ClO⁻, ClO₃⁻, ClO₄⁻, Br, J⁻, JO₃⁻, CN⁻, OCN⁻, NO₂⁻, NO₃⁻, HCO₃⁻, CO₃²⁻ , S²⁻, SH⁻, HSO₃⁻, SO₃²⁻, HSO₄⁻, SO₄²⁻, S₂O₂²⁻, S₂O₄²⁻, S₂O₅²⁻, S₂O₆²-, S₂O₇²⁻, S₂O₈²⁻, H₂PO₂⁻, H₂PO₄⁻, HPO₄²⁻, PO₄³⁻, P₂O₇⁴⁻, (OCₓH₂ₓ₊₁)⁻, (CₓH₂ₓ₋₁O₂)⁻, (CₓH₂ₓ₋₃O₂)⁻, ainsi que (Cₓ₊₁H₂ₓ₋₂O₄)²⁻, x représentant les nombres 1 à 20.

7. Procédé selon l'une quelconque des revendications 1 à 5, **caractérisé en ce que** le composé de bismuth (C) est un carboxylate de bismuth.

8. Procédé selon l'une quelconque des revendications 1 à 5, **caractérisé en ce que** le composé de bismuth (C) est le formiate, l'acétate, le propionate, l'hexanoate, le néodécanoate, le 2-éthylhexanoate, l'octoate ou le pivalate de bismuth.

9. Procédé selon l'une quelconque des revendications 1 à 5, **caractérisé en ce que** le composé de zinc (C) est de niveau d'oxydation +2, avec un anion choisi dans le groupe constitué par F⁻, Cl⁻, ClO⁻, ClO₃⁻, ClO₄⁻, Br⁻, J⁻, JO₃⁻, CN⁻, OCN⁻, NO₂⁻, NO₃⁻, HCO₃⁻, CO₃²⁻, S²⁻, SH⁻, HSO₃⁻, SO₃²⁻, HSO₄⁻, SO₄²⁻, S₂O₂²⁻, S₂O₄²⁻, S₂O₅²⁻, S₂O₆²⁻, S₂O₇²⁻, S₂O₈²⁻, H₂PO₂⁻, H₂PO₄⁻, HPO₄²⁻, PO₄³⁻, P₂O₇⁴⁻, (OCₓH₂ₓ₊₁)⁻, (CₓH₂ₓ₋₁O₂)⁻, (CₓH₂ₓ₋₃O₂)⁻, ainsi que (Cₓ₊₁H₂ₓ₋₂O₄)²⁻, x représentant les nombres 1 à 20.

10. Procédé selon l'une quelconque des revendications précédentes, **caractérisé en ce que** la première étape est réalisée à des températures de 50 à 150 °C pendant une durée de 3 à 48 heures.

11. Procédé selon l'une quelconque des revendications précédentes, **caractérisé en ce que**, lors de la première étape, la stoechiométrie des composants (A) et (B) est de 1:1,5 à 3.

12. Procédé selon l'une quelconque des revendications précédentes, **caractérisé en ce que** le catalyseur (C) est ajouté au mélange réactionnel en quantités de 0,001 à 2 % en poids, par rapport à la somme des composants (A) et (B).

13. Procédé selon l'une quelconque des revendications précédentes, **caractérisé en ce que** la seconde étape est réalisée à 40 à 100 °C.

14. Procédé selon l'une quelconque des revendications précédentes, **caractérisé en ce que** la seconde étape est réalisée à une stoechiométrie de 1,2:1 à 1:1,2 entre les groupes hydroxy dans le produit de réaction de la première étape et les groupes isocyanate dans le composant (D).

15. Utilisation de (méth)acrylates d'uréthane, obtenus par le procédé selon l'une quelconque des revendications précédentes, dans des matériaux de revêtement durcissables par rayonnement.
